(19) Europäisches Patentamt / European Patent Office / Office européen des brevets

(11) **EP 4 205 658 A1**

(12) **EUROPEAN PATENT APPLICATION**

(43) Date of publication:
**05.07.2023 Bulletin 2023/27**

(21) Application number: **22171515.4**

(22) Date of filing: **04.05.2022**

(51) International Patent Classification (IPC):
**A61B 6/03** (2006.01)   **A61B 6/10** (2006.01)
**A61B 6/00** (2006.01)   **H05G 1/54** (2006.01)

(52) Cooperative Patent Classification (CPC):
**A61B 6/032; A61B 6/10; A61B 6/586; G16H 20/30;
G16H 40/40; G16H 40/63; H05G 1/54**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.12.2021   US 202163294087 P**

(71) Applicant: **Koninklijke Philips N.V.
5656 AG Eindhoven (NL)**

(72) Inventors:
• **MILLER, Lester Donald
  Eindhoven (NL)**
• **DAERR, Heiner
  Eindhoven (NL)**
• **RIBBING, Carolina Maria
  Eindhoven (NL)**

(74) Representative: **Philips Intellectual Property &
Standards
High Tech Campus 52
5656 AG Eindhoven (NL)**

(54) **METHOD AND APPARATUS FOR SAFETY MONITORING OF AN IMAGING SYSTEM**

(57)    Method and apparatus for monitoring the operational safety of an imaging system are disclosed. Radiation signals are received directly from one or more radiation detectors. The unattenuated radiation signals and scan metadata data are stored in memory, and time domain analysis and frequency domain analysis is performed on the stored radiation signals. Amplitude and frequency data for all peaks greater than a predetermined Signal to Noise Ratio (SNR) from a spectrum derived from the frequency analysis are recorded. The operational safety of the imaging system is monitored according to the recorded amplitude and frequency data. Previously recorded amplitude and frequency data that is written to a data file may be used to compare against currently recorded amplitude and frequency data or may be used to determine threshold values which when exceed by the currently recorded amplitude and frequency data generate a warning and/or automatic stop of an active scan.

Fig. 3

EP 4 205 658 A1

**Description**

FIELD OF THE INVENTION

The invention relates to the field of medical imaging and, in particular, to a method and an apparatus for monitoring the operational safety of imaging systems.

BACKGROUND OF THE INVENTION

Diagnostic imaging is generally considered noninvasive and safe. While a patient scheduled for an imaging scan may experience some anxiety about the imaging procedure, the patient typically does not expect the imaging system to fail during an imaging procedure. In addition, the patient does not expect to be injured by the imaging system or during the imaging procedure. Although it is not common, there have been a number of instances where patients and/or medical personal have been seriously injured, and in the most severe cases patients have been killed due to a faulty or damaged imaging system. In less severe instances, the imaging system malfunctions during an imaging procedure creating a frightening experience without injuring the patient or medical personal.

Imaging systems such as a CT, CT-arm, Single Photon Emission Computer Tomography CT (SPECT-CT), Magnetic Resonance CT (MR-CT), and Positron Emission Tomography CT (PET-CT) often comprise heavy components rotating at significantly high revolutions per-minute (rpm) and thus carry inherent safety risks due to the high weights and large forces involved with these imaging systems. A typical gantry (rotating component) of a CT scan may rotate over one-thousand pounds at 220 rpm creating significant g-forces on the gantry components. Due to the extreme weight and large g-forces, components and joints soften, crack, become loose, and break. Components may start to loosen, material ageing or fatigue may develop, couplings like welds may start to soften, and imbalances in the imaging system may develop.

The gamma camera portion of a SPECT/CT system can weigh over a thousand pounds. In 2013, bolts holding the 1,300 pound camera to the gantry in a SPECT/CT system came loose, which stressed the support mechanism and resulted in a severe incident where the patient was killed during the exam. The accident led to a Class 1 recall, which was later expanded to nearly all of the manufactures of nuclear imaging systems. In addition to the tragic loss of life, this led to immense costs for both the manufacturer and their customers, not to mention the time and reputation loss.

Incidents such as these stress the importance of continuous safety monitoring of SPECT/CT, CT, CT-arm and related imaging systems. Less severe incidents also occur, for example, tube anode failures like target fracturing, which come with loud explosive and alarming noise, happen. While the tube protective cover is designed to deal with such an event, it is nevertheless frightening for medical personnel and patients and creates a negative user experience.

As a safety precaution, some manufacturers recommend performing a daily scan to observe the rotor balance of the gantry. Other solutions include adding additional sensors and circuits in an attempt to predict the remaining life of imaging system components, and other solutions collect data to in order to perform failure analysis. This would be an example of a reactive rather than predictive approach.

The need exists for a method and apparatus to provide timely warning of safety issues like those mentioned above without the additional burden, added cost, and time to add additional sensors, circuits, and other hardware to the imaging system.

SUMMARY OF THE INVENTION

The object of the present invention provides techniques for safety monitoring of imaging systems with moving parts. The techniques provide a solution for monitor moving and vibrating parts and identifying potential failures before a failure occurs. Certain examples may be employed in a variety of imaging systems that utilize a radiation source, for example an X-ray tube may be a source of radiation. However, the term radiation source and radiation signal may be used to indicate X-ray radiation signals, magnetic fields, radio waves and a combination of magnetic fields and radio waves. Certain examples periodically obtain unattenuated or unfiltered radiation source signals directly at one or more detectors and perform time domain analysis and frequency domain analysis on the radiation source signals to monitor the operation, status, and wear of components of the imaging system and provide timely warning of potential safety issues. Furthermore, the examples do not require any new hardware, which has to be evaluated, certified and funded. Unlike existing monitoring solutions, no additional sensors, components, or circuits are needed. The existing radiation detectors are used as sensor for harvesting a new form of signals without the need for any additional sensors or circuits to provide a timely warning of safety issues like those mentioned above.

According to a first aspect of the invention, an imaging system comprises a stationary part and a rotating part coupled to the stationary part and configured to rotate in relation to the stationary part. A radiation source apparatus is coupled to the rotating part, and one or more radiation detectors are coupled to the rotating part and positioned to receive radiation signals generated by the radiation source apparatus. A motor is configured to rotate the rotating part, and a processor circuit is coupled to the stationary part and configured to obtain unattenuated radiation signals directly from the one or more radiation detectors and obtain scan metadata for an active scan. A memory is configured to store the scan metadata and the obtained radiation signals, and the processor is further configured to: perform time domain analysis and frequency domain analysis of the stored radiation signals; record amplitude and frequency data for all peaks greater than a predetermined Signal to Noise Ratio (SNR) from a spectrum derived from the frequency analysis; and monitor operational safety of the imaging system based on the recorded amplitude and frequency data.

In a second aspect of the invention, a method of monitoring the safety of an imaging system is provided. The method comprising obtaining unattenuated radiation signals directly from one or more radiation signal detectors and obtaining scan metadata for an active scan; storing scan metadata and the obtained radiation signals in memory; performing time domain analysis and frequency domain analysis on the stored radiation signals; recording amplitude and frequency data for all peaks greater than a predetermined Signal to Noise Ratio (SNR) from a spectrum derived from the frequency analysis; and monitoring operational safety of the imaging system based on the recorded amplitude and frequency data.

In a third aspect of the invention, a non-transitory computer-readable medium having stored thereon instructions for causing processing circuitry to execute a process of obtaining unattenuated radiation signals directly from one or more radiation signal detectors and obtaining scan metadata for an active scan; storing scan metadata and the obtained radiation signals in memory; performing time domain analysis and frequency domain analysis on the stored radiation signals; recording amplitude and frequency data for all peaks greater than a predetermined Signal to Noise Ratio (SNR) from a spectrum derived from the frequency analysis; and monitoring operational safety of the imaging system based on the recorded amplitude and frequency data.

It was realized by the inventors that in the process of generating an image of a patient or object under examination, for example a CT image in a CT imaging system, unattenuated or unfiltered radiation signals directly received by a radiation source detector(s) are often used to obtain data that enhance the processing of a patient image. This data is sometimes referred to as correction factor data or calibration data. The data that is collected and applied as a correction factor or as calibration data may be used to monitor the operational health and safety of the imaging system eliminating the need for any external sensors or circuitry to perform safety monitoring. Safety monitoring may be understood to mean identifying a potential failure point in a related imaging system and alerting the operator, owner, or manufacturer of the imaging system of the potential failure prior to the operation of patient imaging, and in some cases, the safety monitoring may be configured to automatically stop a scan when a potential failure is imminent.

As with virtually all electronic devices, in operation, imaging system like at CT system will generate electromagnetic radiation. The signals generated by the electromagnetic radiation are inherently combined with or added to the radiation source signal received at the radiation detectors. Characteristics of the imaging system may be detected in the radiation source signals received at the radiation detectors. For example, during gantry rotation, periodic modulation of the raw or unattenuated radiation may be used to detect potential failure points in the imaging system. The signals added due to the electromagnetic radiation may be referred to as background noise.

In the related imaging systems, the negative effects of noise are especially noticeable. One reason the negative effects of noise are especially noticeable in these systems is the compromise between achieving medically necessary low doses of radiation required for the health and safety of a patient under examination and the behavior of noise when generating images, which increases with the decreasing dose of radiation.

Imaging processing methods are often applied to estimate noise and improve the images generated for medical diagnosis. In order to generate the level of image resolution required by medical professionals, the ability to distinguish between noise and objects under examination is essential. Known methods of estimating noise in related imagining systems include the measurement of unattenuated radiation source signals received at the radiation detectors.

In a patient scan, the unattenuated radiation source signals received at the detectors are collected, and the undesired (noise) portion of the radiation source signal is filtered to provide a higher resolution image of the patient. The same unattenuated radiation source signals received at the detectors to measure the noise of the imaging system may be analyzed to perform safety monitoring and provide a measure of the operational health of the imaging system.

When the rotating part of a related imaging system rotates in relation to the stationary part of the imaging system, the radiation signals received at the radiation detectors change over each rotation due to normal deformation of the system geometry. The changes over each rotation result in modulation of the radiation signals received at the detectors. Any change in the balance of the system is detectable in the radiation signals received at the detectors. Thus, spectral features related to imbalance, deformations, vibrations and the like can be extracted from the unattenuated detector signals and used for monitoring and predicting the operational safety of related imaging systems.

In a preferred embodiment, a process uses the unattenuated radiation signals collected in the normal scan operation of the imaging system to monitor the operational health of the imaging system. The phrase "unattenuated radiation signals" means radiation signals that do not traverse an object or patient under examination. In other words, unattenuated radiation signals are radiation signals that are obtained by various radiation detectors directly from the radiation source.

When a selected scan is performed, the unattenuated radiation signals emitted from the radiation source are obtained by one or more radiation detectors. In a preferred embodiment, the one or more radiation detectors are a reference detector and a patient detector. A patient detector may also be referred to as a Data Measurement System (DMS).

Analysis of the radiation signals may be performed on the unattenuated radiation signals received at the reference detector only; the analysis may be performed on the unattenuated radiation signals received at the patient detector only; and the analysis may be performed on the unattenuated radiation signals received at the reference detector and the patient detector. In an embodiment where analysis of the radiation signals is performed on the unattenuated radiation signals received at the reference detector and the patient detector, the difference between the spectra derived from signals received at the reference detector and the spectra derived from signals received at the patient detector may provide diagnostic information. In other words, it is foreseeable that comparing the spectra derived from the same scan data received at the reference detector and received at the patient detector may reveal disturbances that may not be easily revealed analyzing the radiation signals received at only one of the reference detector and the patient detector.

The techniques for safety monitoring of imaging systems may be performed using one or more scans performed prior to a patient scan, including a daily safety scan performed in the morning and/or the evening, and the techniques provided may be performed during an active patient scan.

In a preferred embodiment, the obtained radiation signals and scan data are stored in a memory component of the imaging system. When an operator performs a scan, scan metadata is collected as part of the image processing. Scan metadata is data that provides information about the scan being performed. In a preferred embodiment, the scan metadata includes at least one of: radiation emission current, scan or shot time, the sampling period, the integration period of the scanned radiation signal, the rotation time, the radiation source acceleration voltage, and the number of samples per rotation.

In a preferred embodiment, the unattenuated radiation signals and scan metadata are obtained from at least one scan selected from the group consisting of existing scans, routine scans generated during normal operation, dedicated scans, and standard scans. Typical scans include an air calibration scan, a scout scan, a helical scan, an axial scan, and a warmup scan. The techniques disclosed may be performed with any type of scan. Analysis taken from a first selected scan may be compared with analysis taken from a second selected scan that is different from the first selected scan. In addition, analysis taken from at least one rotation of a scan may be compared with analysis taken from at least one other rotation of the same scan.

In a preferred embodiment, a processor of the imaging system performs time domain analysis of the radiation signals obtained at the radiation detectors and computes one or more of the mean, median, standard deviation, and range of the detected radiation signal. The processor performs frequency domain analysis to convert the radiation signals from the time domain to the frequency domain, such that a spectrum is derived from the frequency analysis. A time domain graph displays the changes in a signal over a span of time, and the frequency domain displays how much of the signal exists within a given frequency band or spectrum concerning a range of frequencies. A frequency-domain representation can also include information on the phase shift that must be applied to each sinusoid to be able to recombine the frequency components to recover the original time signal.

The frequency domain analysis may be performed using a Fourier transform including a Gabor transform, Wavelet transform, Hilbert-Huang Transform, Bilinear time-frequency distribution, Modified Wingner distribution function, or other known frequency domain analysis methods.

In a preferred embodiment, amplitude data and frequency data for all peaks greater than a predetermined Signal to Noise Ratio (SNR) in the derived spectrum are written to a data file. For example, a SNR greater than 3 may be set to capture 20-40 of the largest peaks, plus their possible occurring harmonics, in the derived spectrum. The predetermined SNR and corresponding number of largest peaks may vary depending on the manufacturer and the design of the imaging system or any number of parameters of the imaging system for which monitoring is desired. In a preferred embodiment, a plurality of amplitudes at plurality of frequencies are obtained for one or more of the selected scans.

In the related imaging systems, most scans are made with the radiation source, the patient detector and/or the reference detector on a rotating gantry. During gantry rotation, the detector signal changes over each rotation due to normal deformation of the system geometry, which results in a modulation of the photon number impinging on the detectors producing a periodic modulation of the raw (unattenuated) radiation signal. Any change in the balance of the system is detectable in the radiation signal.

When imaging system components start to loosen, material ageing or fatigue develops, or connections like welds start to soften, and imbalances will develop. Spectral features related to imbalance, deformations, vibrations and the like can be extracted from unattenuated radiation signals obtained at the detectors and used to predict failures before they occur.

The development of derived spectrum peaks (appearance, disappearance, frequency shift and amplitude changes over time) can be used to monitor system solid mechanics and imbalance. In principle, a developing gantry imbalance will manifest itself as an increase of the gantry rotation peak amplitude, whereas additional gantry wiggle will result in more frequencies in the derived spectrum. In practice a mixture of both will likely be the most common case. Ground frequency harmonics are expected to be a sensitive measure of any balance changes, much like a "fingerprint" for various gantry statuses. Similar reasoning applies to the anode frequency peak and its diagnostic content for impending anode fracture.

The described techniques may constitute a daily safety check in the field. Such a check may be run for each scan. Changes developing in the radiation detector signal derived spectra over time are detected by a comparison to previously derived spectra with the same settings, for example the first and last derived spectra with the same settings, and if changes related to safety are detected, an alert is issued.

In a preferred embodiment, for each of at least one selected scan, the processor is configured to write to a data file the recorded amplitude and frequency data for all peaks greater than the predetermined SNR. The processor is configured to compare the recorded amplitude and frequency data of an active scan with amplitude and frequency data in at least one data file. When a difference between the recorded amplitude and frequency data of the active scan and the amplitude and frequency data in the at least one data file exceeds a predetermined threshold, the processor is configured to generate a warning.

In a preferred embodiment, the processor is configured to write to a data file the recorded amplitude and frequency data for all peaks greater than the predetermined SNR for at least one rotation of a selected scan; compare the recorded amplitude and frequency data for at least one other rotation of the same selected scan with amplitude and frequency data in at least one of the data files; and generate a warning when a difference between the recorded amplitude and frequency data and the amplitude and frequency data in the at least one of the data files exceeds a predetermined threshold. The generated warning may be stopping the scan when the difference indicates an imminent failure.

To simplify the data analysis, it may be preferable that the frequency axis of the derived spectra in the output file should always be the same regardless of the acquisition parameters. The frequency axis $f$ is given by

$$f = 1/(L*T) * (0,1,2,3,...L/2),$$

with T being the averaged integration period (rotation time/number of readings per turn) and $L$ the number of readings including zero padding. The frequency steps are $1/(L*T)$ and the largest frequency is $1/(2*T)$. Typically, T varies depending on the selected rotation time but the frequency steps can be adjusted by increasing $L$ (including zero padding). Setting $1/(L*T) = 0.5$ Hz equals a sampling period of $T*L = 2$ seconds.

After selecting the right number $L = 2sec/T$ of sampling points, the mean of the data should be subtracted and a windowing (Blackman-Harris or Flat Top) should be applied. $L$ does not need to be equal to $2^n$, with n being a non-negative integer, but it needs to be even. In case the signal length is smaller than $L$, zero padding can be applied after mean subtraction and windowing.

After applying the frequency analysis and calculating the amplitudes for the frequencies in $f$ (depending on the actual frequency analysis implementation), the windowing function is corrected for by multiplying with

$$WindowScaleFactor = nData/sum(weights),$$

where nData is the number of data points, and weights are the weights of the windowing function.

For the related imaging systems, suitable radiation detector signals to use for harvesting frequency information may include those resulting from not too short scans (including several (>2) gantry rotations) that are performed regularly and with (nearly) identical settings over time and systems worldwide.

Various scan types and more than one scan type may be used to acquire the radiation signals. Air calibration scans, which are typically performed weekly and would be easy to use in terms of data processing (framework present for air calibration data processing) are one scan type. A protocol with helical movement may be chosen in order to collect the signal from a desired amount of rotation, for example 2-3 gantry rotations per second, with 2-3 second scan time or a protocol with axial movement may be chosen.

First or all long helical scan daily is another candidate scan type. These are typically performed at 120kV and 300-500mA with a rotation time of 0.5-0.2 seconds. Warmup scans such as short-tube conditioning, a scan type that is used to heat up the radiation source or X-ray tube, are typically performed on a daily basis, unless the radiation source or X-ray tube has not been used for an extended duration. Long-tube conditioning, scan type that is used to heat up the X-ray tube, may be used.

Generally, any type of scan at pre-selected settings could be used, for example the first scan of each day at 120kV and 300mA-500mA. It would be advisable to use a scan which is run frequently enough to warrant the required time resolution of the safety status. To provide consistent data, it may be beneficial to use the same kV and mA range and gantry rotation speed, as the g-force F depends quadratically on the rotation speed $\omega$ ($F=m*r*\omega^2$). However, a collection of amplitudes at certain frequencies may be run for each scan type for subsequent comparison with previous scans at the same or similar settings. This would enable real-time monitoring of any component capable of causing disturbances in the radiation detector signals.

An X-ray imaging system may include a single detector or multiple detectors. Related imaging system may include a reference detector and a patient detector or a Data Measurement System (DMS). Each type of detector can be used to collect and measure the radiation signal, and data obtained at both the reference detector and the patient detector may be used to perform the safety monitoring analysis.

The disclosed techniques use an unattenuated or unfiltered radiation signal that does not traverse a patient or object. Since the radiation signals that traverse a patent or object are altered by the patient or object under examination, the unattenuated or unfiltered radiation signals received directly at the radiation signal detectors are preferred for performing analysis.

A reference detector receives radiation signals directly from the radiation source. A DMS/patient detector is typically positioned such that the radiation signals traverse a patient or object. However, a certain slice or portion of the radiation signals received by the DMS/patient detector does not traverse a patient or object of interest and can be used to directly receive unattenuated radiation signals.

In another aspect of the invention, the unattenuated radiation signals obtained directly from the detectors and the scan metadata are written to a data file. In a preferred embodiment, analysis of the radiation signals and scan metadata file is performed with machine learning or deep learning techniques, the machine learning or deep learning techniques including at least one of neural networks, logistic regression, random forests, nearest neighbors, and cluster or multivariate analysis.

In another aspect, data from other sources is optionally included. For example, data from extra sensors, log data from the radiation source or X-ray tube, host computer, system, manufacturing, or shipping data may be combined with the stored radiation signals and the scan metadata. Time domain and frequency domain analysis may be performed with the optionally included data form other sources. Alternatively, the optionally included data form other sources may be combined with the recorded amplitude and frequency data for all peaks greater than a predetermined threshold in the spectrum derived from the frequency analysis.

In one aspect, trained machine learning or deep learning models, trained with data from other systems, may be applied to derive predictions from the obtained unattenuated signals in time or frequency domain and the metadata directly. In another aspect, trained machine learning or deep learning models, trained with data from other systems, may be applied to derive predictions using extracted features like the largest peaks plus their possible occurring harmonics. In other words, in one approach the machine gets all available data and learns the features itself during the training or deep learning phase, and in another approach a user provides features extracted from the available data and trains a model based on these features.

## BRIEF DESCRIPTION OF THE DRAWINGS

In the following drawings:

Fig. 1 is a diagram of a radiation imaging system apparatus;
Fig. 2 is a block diagram according to some embodiments;
Fig. 3 is a block diagram according to some embodiments;
Fig. 4 is a flowchart representing a method of monitoring the safety of an imaging system according to some embodiments;
Fig. 5 is a flowchart representing a method of monitoring the safety of an imaging system according to some embodiments;
Fig. 6 illustrates a radiation signal and corresponding derived spectrum;
Fig. 7 illustrates a radiation signal and corresponding derived spectrum according to some embodiments; and
Fig. 8 illustrates a radiation signal and corresponding derived spectrum according to some embodiments.

## DETAILED DESCRIPTION OF EMBODIMENTS

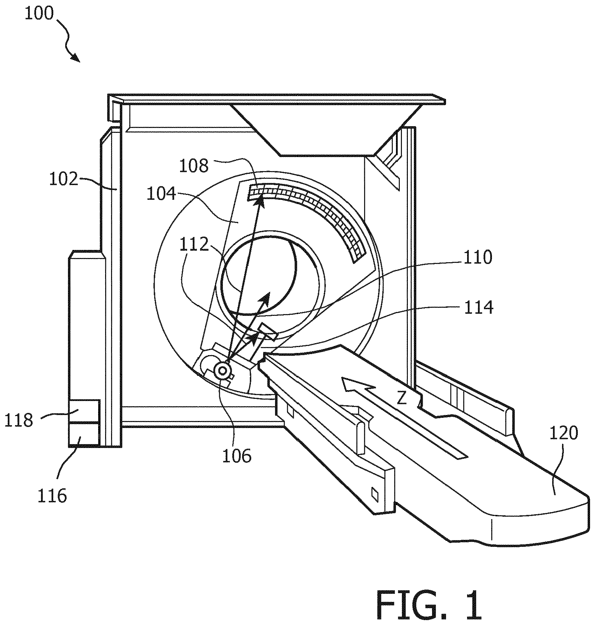

Fig. 1 shows a radiation imaging system, which in this case is an example of a Computed Tomography (CT) imaging system 100. CT device 100 includes a stationary part 102 and a rotating part 104 that rotates in relation to the stationary part 102. A radiation source apparatus 106 is positioned at the rotating part 104, and across from the radiation apparatus 106 are positioned radiation detector elements 108 which receive radiation signals 110 and 112 emitted from the radiation source apparatus 106. It is noted that radiation signals 110 and 112 are the same radiation signals. Radiation signals 110 are used to indicate radiation signals that are attenuated by a patient or object under examination (not shown), while radiation signals 112 are used to illustrate the radiation signals that are not attenuated by a patient or object under examination. Radiation signals 112 are also received directly at radiation detector 114. The radiation signals 110 traverse an examination subject (not shown), for example a patient, during an examination. When radiation signals 110 traverse the examination subject, they are attenuated or filtered by the examination subject (not shown) and received at detector elements 108. Preferably, the unattenuated or unfiltered radiation signals 112 received directly at the radiation signal detectors are used for analysis, because radiation signals 110 are altered by the patient under examination and add complexity to the analysis.

Peripheral detector elements of radiation detector elements 108 may also receive radiation signals 112 directly from the radiation source apparatus. In other words, in addition to being received directly at reference detector 114, radiation signals 112 that do not traverse the examination subject may also be received at peripheral detector elements of detector elements 108. Thus, radiation signals 112 may be obtained from the reference detector 114 and detector elements of radiation detector elements 108. The unattenuated or unfiltered radiation signals 112 may be referred to as raw radiation signals. The imaging system apparatus also includes processor circuitry 116 and memory 118.

When operated, the rotating part 104 rotates in relation to the stationary part 102 around the examination subject. During an examination, data processing circuitry (not shown) creates multiple CT images of the examination subject by known means of processing the spatial variances in radiation signals 110 that are attenuated or filtered by traversing the subject under examination and received at detector elements 108.

Processor circuitry 116 obtains the unattenuated radiation signals 112 directly from one or more detectors 108 and 114 and also obtains scan metadata that is generated for each scan being performed. The obtained radiation signals and metadata are stored in memory 118. Processor circuitry 116 performs time domain analysis and frequency domain analysis of the stored radiation signals, records amplitude and frequency data for all peaks greater than a predetermined Signal to Noise Ratio (SNR) from a spectrum derived from the frequency analysis, and monitors the operational safety of the imaging system based on the recorded amplitude and frequency data. In addition, safety scans may be performed prior to a patient scan.

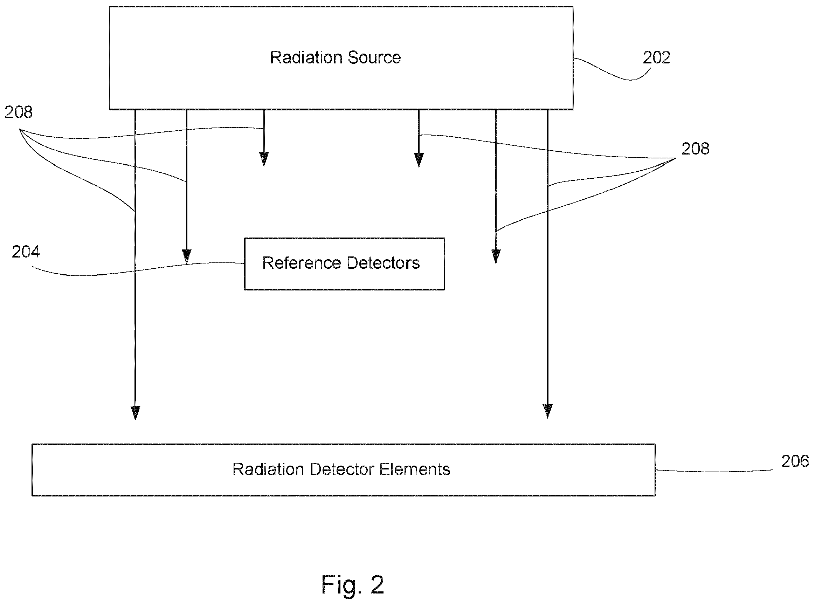

Fig. 2 is a block diagram according to some embodiments. A safety scan may be performed prior to a patient imaging scan. Thus, a type of safety scan may be performed before a patient is placed between the radiation source 202 and the radiation detector elements 206. In a safety scan, radiation signals 208 are emitted by a radiation source 202. Radiation signals 208 are received at reference detectors 204 and radiation detector elements 206.

In a preferred embodiment, recorded amplitude and frequency data for at least one rotation of a selected scan is written to a data file, amplitude and frequency data for at least one other rotation of the same scan is recorded and compared to the amplitude and frequency data in the data file, and a warning is generated when a difference between the recorded amplitude and frequency data and the amplitude and frequency data in the data file exceeds a predetermined threshold.

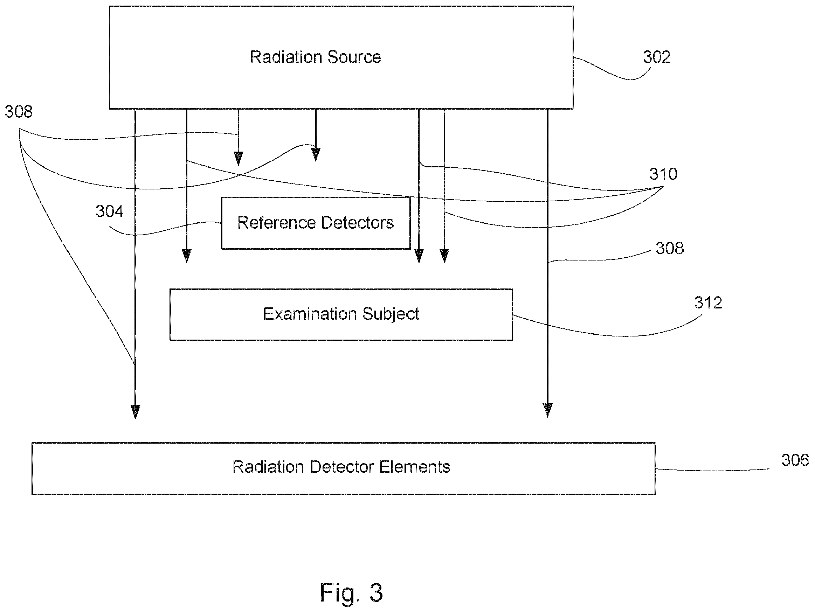

Fig. 3 is a block diagram according to some embodiments. Radiation signals 308 and 310 are emitted by radiation source 302. Radiation signals 308 are received at reference detectors 304 and radiation detector elements 306. Radiation signals 310 traverse examination subject 312 and are attenuated or filtered by the examination subject 312 before being received at radiation detector elements 306. Radiation signals 308 are received at peripheral detector elements of radiation detector elements 306 and are received directly at reference detectors 304. Radiation detector elements 306 may be referred to as a patient detector or as a Data Measurement System (DMS).

In a patient scan, the unattenuated radiation signals 308 received at radiation detectors 304 and 306 are collected, and the undesired (noise) portion of the radiation signal is filtered to provide a higher resolution image of the patient. The same unattenuated X-ray radiation signals 308 received at the radiation detectors 304 and 306 to measure the noise of the imaging system may be analyzed to provide an account of the operational safety of the imaging system.

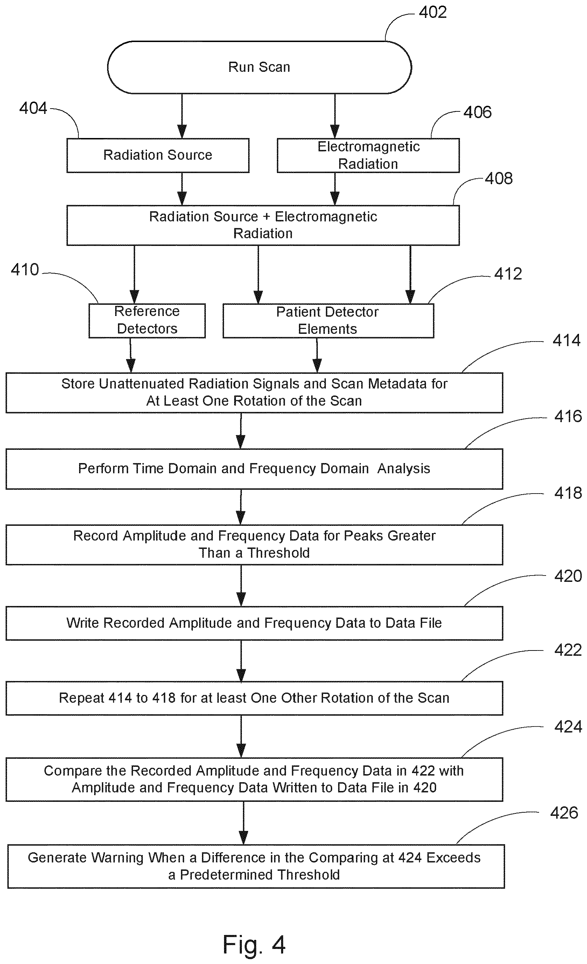

Fig. 4 is a flowchart representing a method of monitoring the operational safety of an imaging system. In an aspect of the invention, a safety scan may be performed prior to performing an imaging scan of a patient.

At block 402 a safety scan is started. At block 404, radiation signals are generated. At block 406, electromagnetic radiation from the imaging system is generated due to the underlying electrical components of the imaging system.

At block 408 radiation signals generated at 404 and electromagnetic radiation signals generated at 406 are combined. At block 410 the combined signals are received directly at a reference detector, and at block 412 the combined signals are received at patient detector elements. During gantry rotation, the radiation signals received at the detector elements (reference and patient detectors) change over each rotation due to normal deformation of the system geometry. This change results in a modulation of the photon number impinging on the detector elements which causes a periodic modulation of the raw or unattenuated radiation signal.

At block 414 scan metadata and the radiation signals directly received at the reference detector and/or the radiation signals received directly at peripheral elements of the patient detector elements for at least one rotation of a scan are stored in memory. At block 416 time domain analysis and frequency domain analysis are performed on the stored scan metadata and radiation signals. At block 418 amplitude data and frequency data for all peaks greater than threshold are recorded. At block 420 the recorded amplitude and frequency data are written to a data file. At block 422, the process in blocks 414 to 418 is repeated for at least one other rotation of the same scan. At block 424, the recorded amplitude and frequency data of block 422 is compared with the amplitude and frequency data written to a data file in block 420. At block 426, when a difference between the recorded amplitude and frequency data of block 422 and the amplitude and frequency data written to a data file in block 420 exceeds a predetermined threshold a warning and/or stop command is generated.

The process illustrated in Fig. 4 may be performed as a safety scan shortly before beginning an imaging scan of a patient. A safety scan may be performed where time domain and frequency domain analysis is performed on one or several rotations of a selected scan and the recorded amplitude and frequency data written to a data file. Time domain and frequency domain analysis is then performed on one or several additional rotations of the same selected scan and compared to the time domain and frequency domain analysis in the data file.

This process allows a comparison within a scan. By performing the analysis for each rotation or a couple of rotations, frequencies of a few Hz and smaller may be observed and amplitude changes during the scan may be monitored. In addition, this allows spectrum generation of a moving time window with analysis of how it changes over time. If a critical increase of amplitudes is observed, the system may be stopped automatically. While the process illustrated in Fig. 4 is described as a safety scan performed prior to a patient imaging scan, the process may also be performed concurrent with a patient imaging scan.

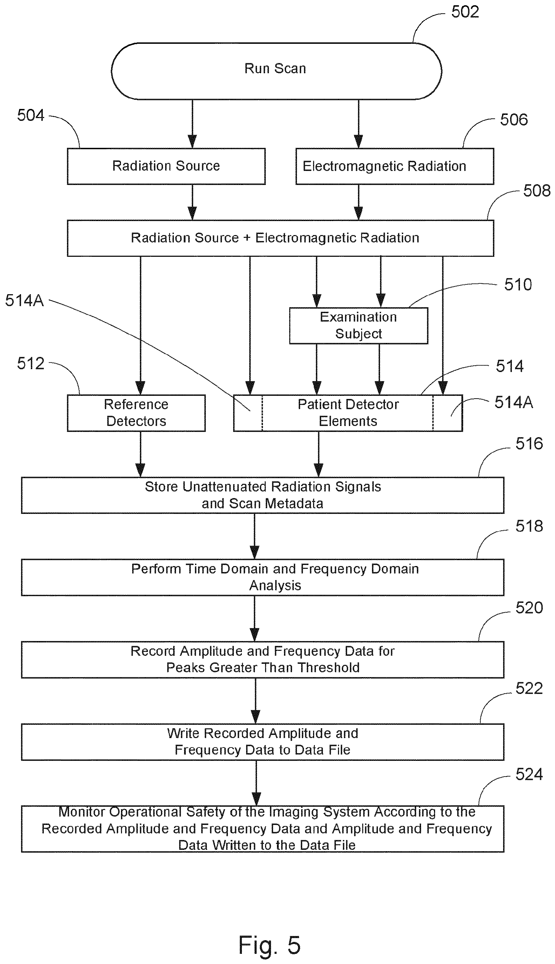

Fig. 5 is a flowchart representing a method of monitoring the operational safety of an imaging system. At block 502 an imaging scan is started. At block 504, radiation signals are generated. At block 506, electromagnetic radiation from the imaging system is generated due to the underlying electrical components of the imaging system.

At block 508 radiation signals generated at 504 and electromagnetic radiation signals generated at 506 are combined. At block 510 an examination subject attenuates the combined signals. At block 512 the combined signals are received directly at a reference detector. At block 514 the combined signals attenuated by the examination subject are received at patient detector elements. At block 514A the combined signals are received directly at peripheral elements of the patent detector elements without being attenuated by the examination subject.

At block 516 scan metadata and the combined signals received directly at the reference detector and/or the combined signals received directly at peripheral elements of the patient detector elements are stored in memory. At block 518 time domain analysis and frequency domain analysis is performed on the stored scan metadata and combined signals. At block 520 amplitude data and frequency data for all peaks greater than threshold are recorded. At block 522 the recorded amplitude and frequency data are written to a data file. The operational safety of the imaging is monitored at block 524.

In one aspect of the invention, the recorded amplitude and frequency data is compared to amplitude and frequency data in at least one of the data files of block 522. When a difference between the recorded amplitude and frequency data exceeds a predetermined threshold a warning and/or stop command is generated.

In another aspect of the invention, the amplitude and frequency data in at least one of the data files at 522 is used to configure a predetermined threshold. When the recorded amplitude and frequency data exceeds the configured predetermined threshold, a warning and/or stop is generated.

In yet another aspect of the invention, the unattenuated radiation signals and metadata stored at block 516 is written to a data file and the analysis of the scan metadata and the radiation signals is performed with machine learning or deep learning techniques, the machine learning or deep learning techniques including at least one of neural networks, logistic regression, random forests, nearest neighbors, and cluster or multivariate analysis.

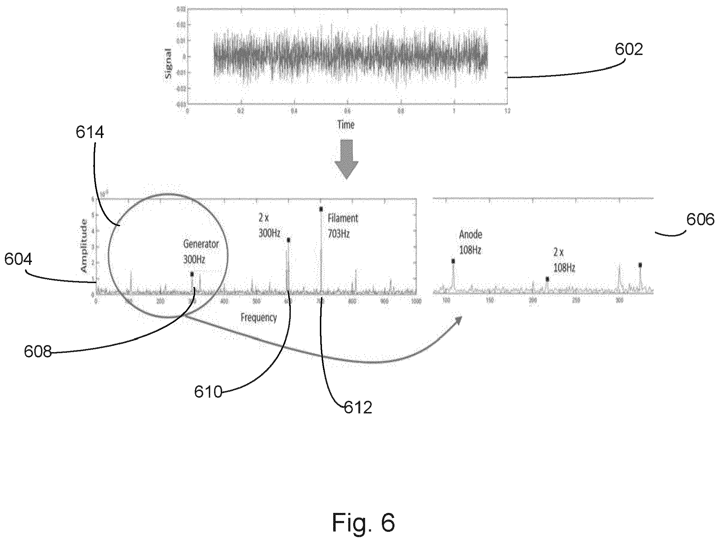

Fig. 6 is a graphical representation of a radiation signal analyzed without gantry rotation. 602 illustrates an unattenuated radiation signal obtained during a scan performed on a Philips MRC600 rotating at 108Hz. The scan is performed without gantry rotation. 604 illustrates a 0 to 1000Hz spectrum derived from the frequency analysis. 606 illustrates approximately 0 to 400Hz spectrum derived from the frequency analysis. The spectrum illustrated in 604 and the spectrum illustrated in 606 are both derived from the radiation signal obtained at 602. It can be seen that the spectrum illustrated in 606 is the spectrum identified in 614.

The signals 608 and 610 at 300Hz and 600Hz, respectively, are from the generator power, and the signal 612 at 703Hz is potentially from filament vibration. Because the scan illustrated in Fig. 6 is made without gantry rotation, there is no resulting periodic modulation of the radiation signal.

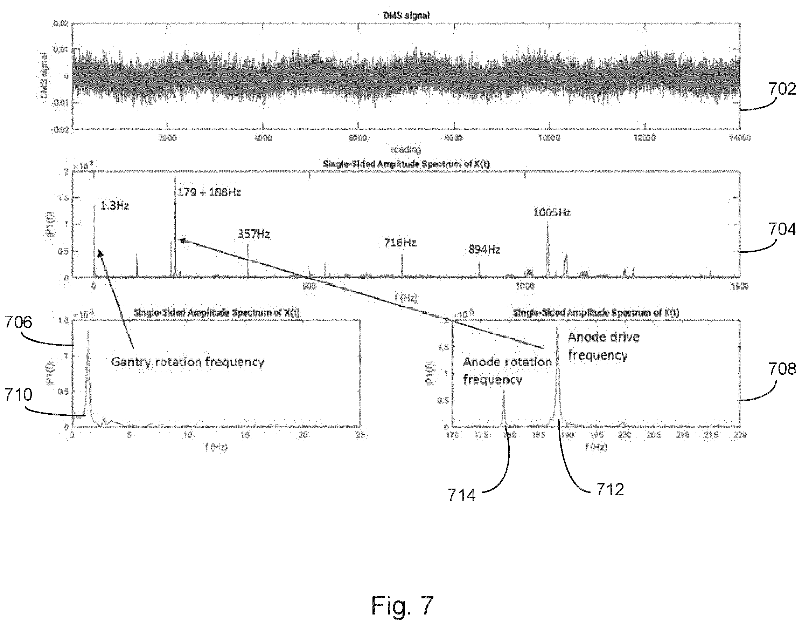

Fig. 7 is a graphical representation of a radiation signal analyzed according to some embodiments. 702 illustrates an unattenuated radiation signal from an intraoperative CT (iCT) with a rotating gantry. The gantry rotation is visible as radiation signal modulation in 702. 704 illustrates the derived power spectrum of the unattenuated radiation signal 702. 706 illustrates a localized view of the gantry rotation with a frequency peak 710 at 1.3Hz, and 708 illustrates a localized view of two anode rotation frequency peaks 712 and 714. Unlike the spectrum derived from a scan without gantry rotation illustrated in Fig. 6, the slip between anode drive frequency 712 at 188Hz and the anode rotation frequency 714 at 179Hz can be seen.

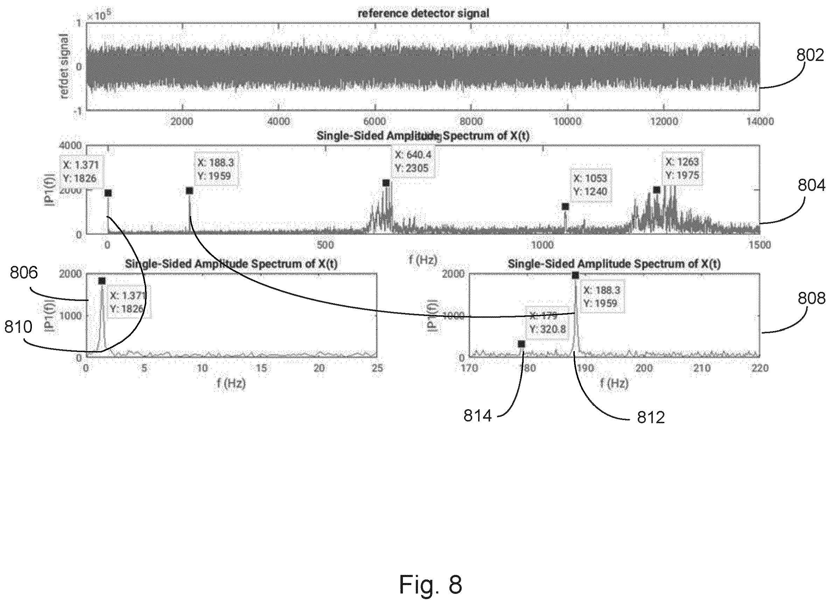

Fig. 8 is a graphical representation of a radiation signal analyzed according to some embodiments. 802 illustrates an unattenuated radiation signal sampled at 3200 samples per second (Nyquist frequency of 1600Hz) from an iCT with a gantry rotating at 1.33Hz. 804 illustrates the derived power spectrum of the unattenuated radiation signal 802. 806 illustrates a localized view of the gantry rotation with a frequency peak 810 at 1.37Hz, and 808 illustrates a localized view of two anode rotation frequency peaks 812 and 814, the anode drive frequency 812 at 179Hz and anode rotation frequency 814 at 188Hz.

In the spectrum derived from the raw or unattenuated radiation signal, amplitude and frequency are plotted. The amplitude reflects the relative strength of the signal at a given frequency. In the frequency analysis a signal to noise ratio (SNR) may be set such that the amplitude of peaks greater than a predetermined SNR may be recorded. The amplitude is plotted on the y-axis and the frequency is plotted on the x-axis. As an example, reference numeral 810 points to an illustration of x, y coordinates of 1.371Hz and a relative amplitude of 1826, respectively.

Certain frequency "fingerprints," identified by their respective x, y coordinates, may be used to monitor the safety of an imaging system with moving parts. In a preferred embodiment, the recorded amplitude and frequency data for all peaks greater than a predetermined SNR from the spectrum derived from the frequency analysis are compared to a threshold range. When the recorded amplitude data and the recorded frequency data for a given component, taken alone or in combination, appear outside of the threshold range, the processor circuitry of the imaging system may be configured to notify the system operator of a potential failure in real time.

In a preferred embodiment, the recorded amplitude and frequency data for all peaks greater than the predetermined SNR are written to a data file. The data files may be viewed as a collection of component "fingerprints." When a scan is performed, the recorded "fingerprints" derived from an active scan may be compared to corresponding "fingerprints" in one or more data files. When a difference between the recorded "fingerprints" derived from the active scan deviate and the corresponding "fingerprints" in one or more data files exceed a predetermined threshold or limit, the imaging system may generate a warning and/or may automatically stop the active scan.

In another aspect, the fingerprints" in one or more data files may be used to configure a predetermined threshold such that the recorded "fingerprints" derived from an active scan may be compared to the predetermined threshold. When the predetermined threshold is exceeded during the same active scan, the imaging system may generate a warning and/or may automatically stop the active scan.

In the derived spectrum, features related to imbalance, deformations, vibrations and the like can be extracted from the unattenuated radiation signals received at the detectors and used for predicting, monitoring, and avoiding failure. The development of peaks (appearance, disappearance, frequency shift and amplitude changes over time) in the derived spectrum may be used to monitor system solid mechanics and imbalance. In principle, a developing gantry imbalance will manifest itself as an increase of the gantry rotation peak amplitude, and additional gantry wiggle will result in more frequencies in the derived spectrum. However, in practice a mixture of amplitude peak and frequency variation will likely be most common. Ground frequency harmonics are considered to be a sensitive measure of any balance changes, much like a fingerprint for various gantry statuses. A similar reasoning applies to the anode frequency peak and its diagnostic content for impending anode fracture.

While the invention has been illustrated and described in detail in the drawings and foregoing description, such illustrations and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single processor, device or other unit may fulfill the functions of several items recited in the claims. The mere fact that certain measures are recited in mutually different dependent claims does not indicate that a combination of these measures cannot be used to advantage.

Operations like acquiring, determining, obtaining, outputting, providing, store or storing, calculating, simulating, receiving, warning, and stopping can be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. An imaging system comprising:

   a stationary part;
   a rotational part coupled to the stationary part and configured to rotate in relation to the stationary part;
   a radiation source apparatus coupled to the rotational part;
   one or more radiation detectors coupled to the rotational part and positioned to receive radiation signals generated by the radiation source apparatus;
   a motor configured to rotate the rotational part;
   a processor circuit coupled to the stationary part and configured to obtain unattenuated radiation signals directly from the one or more radiation detectors obtain scan metadata for an active scan; and
   a memory configured to store the scan metadata the obtained radiation signals and,

   wherein the processor is configured to:

perform time domain analysis and frequency domain analysis of the stored radiation signals; record amplitude and frequency data for all peaks greater than a predetermined Signal to Noise Ratio (SNR) from a spectrum derived from the frequency analysis; and monitor operational safety of the imaging system based on the recorded amplitude and frequency data.

2. The imaging system according to claim 1, wherein the one or more radiation detectors are at least a radiation reference detector and a patient detector, and wherein the unattenuated radiation signals and scan metadata are obtained from at least one scan selected from the group consisting of existing scans, routine scans generated during normal operation, dedicated scans, and standard scans.

3. The imaging system according to claim 2, wherein a plurality of amplitudes at plurality of frequencies are obtained for one or more of the selected scans.

4. The imaging system according to claim 1, wherein the scan metadata includes at least one of: radiation source apparatus emission current, scan or shot time, sampling period, integration period of the obtained radiation signal, rotation time, radiation source apparatus acceleration voltage, and number of samples per rotation.

5. The imaging system according to claim 2, wherein, for each of the at least one selected scan, the processor is further configured to write to a data file the recorded amplitude and frequency data for all peaks greater than the predetermined SNR.

6. The imaging system according to claim 5, wherein the processor is configured to compare the recorded amplitude and frequency data with amplitude and frequency data in at least one said data file.

7. The imaging system according to claim 6, wherein the processor is configured to generate a warning when a difference between the recorded amplitude and frequency data and the amplitude and frequency data in the at least one said data file exceeds a predetermined threshold.

8. The imaging system according to claim 2, wherein the processor is configured to:

   write to a data file the recorded amplitude and frequency data for all peaks greater than the predetermined SNR for at least one rotation of the selected scan; compare the recorded amplitude and frequency data for at least one other rotation of the same selected scan with amplitude and frequency data in at least one said data file; and generate a warning and/or automatic stop when a difference between the recorded amplitude and frequency data and the amplitude and frequency data in the at least one said data file exceeds a predetermined threshold.

9. A method of monitoring safety of an imaging system, the method comprising:

   obtaining unattenuated radiation signals directly from one or more radiation signal detectors; obtaining scan metadata for an active scan; storing scan metadata and the obtained radiation signals in memory; performing time domain analysis and frequency domain analysis on the stored radiation signals; recording amplitude and frequency data for all peaks greater than a predetermined Signal to Noise Ratio (SNR) from a spectrum derived from the frequency analysis; and monitoring operational safety of the imaging system based on the recorded amplitude and frequency data.

10. The method according to claim 9, wherein the unattenuated radiation signals are obtained directly from at least one of a radiation signal reference detector and a patient detector, and wherein the unattenuated radiation signals and scan metadata are obtained from at least one scan selected from the group consisting of existing scans, routine scans generated during normal operation, dedicated scans, and standard scans.

11. The method according to claim 10, wherein a plurality of amplitudes at plurality of frequencies are obtained for one or more of the selected scans.

12. The method according to claim 9, wherein the scan metadata includes at least one of: radiation source apparatus emission current, scan or shot time, sampling period, integration period of the obtained unattenuated radiation signal, rotation time, radiation source apparatus acceleration voltage, and number of samples per rotation.

13. The method according to claim 11, further comprising writing to a data file the recorded amplitude and frequency data for all peaks greater than the predetermined SNR for each of the at least one selected scan.

14. The method according to claim 13, further comprising comparing the recorded amplitude and frequency data with amplitude and frequency data in at least one said data file.

**15.** A non-transitory computer-readable medium having stored thereon instructions for causing processing circuitry to execute a process of monitoring safety of an imaging system, the process comprising:

obtaining scan metadata and unattenuated radiation signals directly from one or more radiation signal detectors;
obtaining scan metadata for an active scan;
storing scan metadata and the obtained radiation signals in memory;
performing time domain analysis and frequency domain analysis on the stored radiation signals;
recording amplitude and frequency data for all peaks greater than a predetermined Signal to Noise Ratio (SNR) from a spectrum derived from the frequency analysis;
writing the recorded amplitude and frequency data for all peaks greater than the predetermined SNR to a data file; and
monitoring operational safety of the imaging system based on the recorded amplitude and frequency data and the amplitude and frequency data in at least one said data file.

FIG. 1

Fig. 2

Fig. 3

Fig. 4

502

Run Scan

504

Radiation Source

506

Electromagnetic Radiation

508

Radiation Source + Electromagnetic Radiation

514A

510

Examination
Subject

512

514

Reference
Detectors

Patient Detector
Elements

514A

516

Store Unattenuated Radiation Signals
and Scan Metadata

518

Perform Time Domain and Frequency Domain
Analysis

520

Record Amplitude and Frequency Data for
Peaks Greater Than Threshold

522

Write Recorded Amplitude and
Frequency Data to Data File

524

Monitor Operational Safety of the Imaging System According to the
Recorded Amplitude and Frequency Data and Amplitude and Frequency
Data Written to the Data File

Fig. 5

Fig. 6

Fig. 7

Fig. 8

EP 4 205 658 A1

Europäisches
Patentamt

European
Patent Office

Office européen
des brevets

# EUROPEAN SEARCH REPORT

Application Number

EP 22 17 1515

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/172854 A1 (MOORE JARED WILLIAM [US]) 21 June 2018 (2018-06-21) * paragraphs [0032], [0033], [0040], [0057], [0059], [0061], [0063], [0066]; figures 3,10 * ----- | 1-15 | INV. A61B6/03 A61B6/10 A61B6/00 H05G1/54 |
| X | DE 10 2011 075804 A1 (SIEMENS AG [DE]) 15 November 2012 (2012-11-15) * paragraphs [0008], [0031], [0046]; figure 5 * ----- | 1,9,15 | |

|  |  |
|---|---|
|  | TECHNICAL FIELDS SEARCHED (IPC) |
|  | A61B H05G |

The present search report has been drawn up for all claims

| Place of search | Date of completion of the search | Examiner |
|---|---|---|
| Munich | 19 October 2022 | Koprinarov, Ivaylo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

**EP 4 205 658 A1**

## ANNEX TO THE EUROPEAN SEARCH REPORT
## ON EUROPEAN PATENT APPLICATION NO.

EP 22 17 1515

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2022

| Patent document cited in search report | | Publication date | Patent family member(s) | | Publication date |
|---|---|---|---|---|---|
| US 2018172854 | A1 | 21-06-2018 | CN | 110113995 A | 09-08-2019 |
| | | | EP | 3554372 A1 | 23-10-2019 |
| | | | PL | 3554372 T3 | 20-12-2021 |
| | | | US | 2018172854 A1 | 21-06-2018 |
| | | | WO | 2018112039 A1 | 21-06-2018 |
| DE 102011075804 | A1 | 15-11-2012 | BR | 102012011433 A2 | 18-06-2013 |
| | | | CN | 102772218 A | 14-11-2012 |
| | | | DE | 102011075804 A1 | 15-11-2012 |

EPO FORM P0459

For more details about this annex : see Official Journal of the European Patent Office, No. 12/82